# EUROPEAN PATENT APPLICATION

(11) **EP 4 624 495 A1**
(43) Date of publication of application: **01.10.2025**
(21) Application number: 23894919.2
(22) Date of filing: 16.11.2023
(51) Int. Cl.: C07K 16/40, A61P 25/28, A23L 33/18, A61K 39/00

(54) **ANTIBODY BINDING SPECIFICALLY TO ASM PROTEIN**

(30) Priority: 25.11.2022 KR 20220160443
(71) Applicant: Isu Abxis Co., Ltd., Seongnam-si, Gyeonggi-do 13488 (KR)
(72) Inventor: CHO, Kyueun, Seongnam-si, Gyeonggi-do 13488 (KR); CHO, Jeongin, Seongnam-si, Gyeonggi-do 13488 (KR)
(74) Representative: Maiwald GmbH
(86) International application number: PCT/KR2023/018407
(87) International publication number: WO 2024/111990

(57) **Abstract**

The present disclosure relates to an antibody specifically binding to an acid sphingomyelinase (ASM) protein and, specifically, the antibody or antigen-binding fragment according to the present disclosure specifically binds to an ASM protein with high affinity and thus can be used in detecting the ASM protein or diagnosing a disease occurring due to the over-expression of the ASM protein.

## Description

### [Technical Field]

The present disclosure relates to antibodies specifically binding to acid sphingomyelinase (ASM).

### [Background Art]

Sphingolipid metabolism regulates normal cellular signaling, and abnormal changes in sphingolipid metabolism cause various neurodegenerative diseases including Alzheimer's disease. Acid sphingomyelinase (ASM) protein, an enzyme that regulates sphingolipid metabolism, is a protein expressed in almost all types of cells and plays an important role in sphingolipid metabolism and cell membrane turnover.

In the brain of patients with neurodegenerative diseases including Alzheimer's disease, the activity of ASM protein is significantly increased compared with that of normal people. In this connection, Korean Patent No. 10-1521117 discloses that the inhibition of the activity of over-expressed ASM protein or the inhibition of the expression into ASM protein suppresses the accumulation of amyloid-β and improves learning ability and memory and thus can treat neurodegenerative diseases. It has been recently known that the activity of ASM protein is also increased in neurological diseases, such as depression, and thus the inhibition of the expression or activity of ASM protein is effective in the amelioration of depression.

However, substances that directly inhibit the expression or activity of ASM protein have not been developed, and several types of inhibitors that indirectly inhibit the expression of ASM proteins have been identified. For example, there are tricyclic antidepressants used in the treatment of depression, and examples thereof include amitriptyline, desipramine, mipramine, and the like. Although these tricyclic antidepressants were not developed as ASM protein inhibitors, various studies have demonstrated that such antidepressants exhibit ASM protein inhibitory effects. A major pharmacological mechanism of tricyclic antidepressants is that the activity thereof is increased by inhibiting the reuptake of neurotransmitters in nerve cells, and their action as an ASM inhibitor was confirmed to be a subordinate action. However, tricyclic antidepressants may act on the nervous system and nerve cells, causing side effects, such as blurred vision, increased light sensitivity, and vomiting.

### [Technical Problem]

Therefore, an aspect of the present disclosure is to provide an antibody or antigen-binding fragment thereof that specifically binds to ASM protein.

Another aspect of the present disclosure is to provide a method for producing the antibody or antigen-binding fragment thereof.

Still another aspect of the present disclosure is to provide use of the antibody or antigen-binding fragment thereof for detecting ASM protein.

### [Technical Solution]

In accordance with aspects, the present disclosure provides an antibody or antigen-binding fragment thereof that specifically binds to an acid sphingomyelinase (ASM) protein.

Furthermore, the present disclosure provides a nucleic acid encoding the antibody or antigen-binding fragment thereof.

Furthermore, the present disclosure provides an expression vector including the nucleic acid.

Furthermore, the present disclosure provides a host cell including the nucleic acid or expression vector.

Furthermore, the present disclosure provides a method for producing an antibody or antigen-binding fragment thereof that specifically binds to an ASM protein, the method including culturing the host cell to produce the antibody or antigen-binding fragment thereof.

Furthermore, the present disclosure provides a composition and kit for detecting an ASM protein, each including the antibody or antigen-binding fragment thereof.

Furthermore, the present disclosure provides a method for detecting an ASM protein, the method including reacting a sample with the antibody or antigen-binding fragment thereof.

### [Advantageous Effects]

The antibody or antigen-binding fragment according to the present disclosure specifically binds to an ASM protein with high affinity and thus can be used in detecting the ASM protein or diagnosing a disease occurring due to the over-expression of the ASM protein.

### [DETAILED DESCRIPTION OF THE EXEMPLARY EMBODIMENTS]

Hereinafter, the present disclosure will be described in detail.

The present disclosure provides an antibody or antigen-binding fragment thereof that specifically binds to an acid sphingomyelinase (ASM) protein.

As used herein, the term "acid sphingomyelinase (ASM) protein" refers to an enzyme, which is a member of the sphingomyelinase (SMase) family that regulates sphingolipid metabolism. The ASM protein catalyzes the degradation of sphingomyelin into ceramide and phosphorylcholine and may be classified as an alkaline, neutral, or acidic ASM protein depending to pH at which optimal enzymatic activity thereof is exhibited.

The ASM protein may encompass any type of ASM protein that is known in the art. Specifically, the ASM protein may be derived from a mammal, more specifically, a human, a monkey, a rat, or a mouse. In addition, the ASM protein may contain all the amino acid sequences known as the ASM protein in the art. For example, the ASM protein may be a polypeptide consisting of the amino acid sequences as set forth in SEQ ID NO: 139, or a nucleic acid encoding the same.

The ASM protein may be one that has addition, deletion, or substitution of at least one amino acid in the amino acid sequence as set forth in SEQ ID NO: 139 as long as one retains biological activity equivalent or corresponding to that of the ASM protein. The substitution of the amino acid may be conservative substitution that is performed within a range in which the substitution does not affect or gives a weak effect on the entire protein charge, that is, polarity or hydrophobicity. The ASM protein may also have at least 80%, at least 90%, at least 95%, at least 97%, or at least 99% homology with the amino acid sequences as set forth in SEQ ID NO: 139.

As used herein, the term "antibody" refers to an immune protein that binds to an antigen to interfere with an action of the antigen or eliminate the antigen. The antibody may include all types of antibodies known in the art. Specifically, the antibody may include IgM, IgD, IgG, IgA, and IgE, which may contain heavy chains formed from µ, δ, γ, α, and ε, genes encoding heavy chain constant regions, respectively. In antibody technology, IgG is typically used. IgG may include an isotype, IgG1, IgG2, IgG3, or IgG4, which may be different in terms of structural and functional characteristics. The antibody may include all of a humanized antibody containing a minimal sequence derived from a non-human antibody, a human antibody containing a sequence derived from a human, or a chimeric antibody containing mixed sequences derived from different species.

The IgG may have a very stable Y-shaped structure (about 150 kDa) made of two heavy chain proteins of about 50 kDa and two light chain proteins of about 25 kDa. Heavy and light chains constituting antibodies may be divided into variable regions having amino acid sequences, which are different among antibodies, and constant regions having amino acid sequences, which are same among antibodies. The heavy chain constant regions include CH1, hinge (H), CH2, and CH3 domains, each of which is composed of two β-sheets and which may be linked via an intermolecular disulfide bond. In addition, two heavy chain and light chain variable regions are combined to form an antigen-binding site, and one antigen binding site may be present in each of the two Y-shaped arms. In the full-length antibody, a region that can bind to an antigen is called an antibody binding fragment (Fab), and a region that cannot bind to an antigen is called a crystallizable fragment (Fc), and the Fab and Fc may be connected by a hinge.

In an embodiment of the present disclosure, the IgG may be a human-derived IgG and, specifically, may include a human IgG heavy chain constant region consisting of the amino acid sequence as set forth in SEQ ID NO: 137 and a human light chain λ constant region consisting of the amino acid sequence as set forth in SEQ ID NO: 138. Alternatively, the human-derived IgG may include a human IgG heavy chain constant region consisting of the nucleotide sequence as set forth in SEQ ID NO: 140 and a human light chain λ constant region consisting of the nucleotide sequence as set forth in SEQ ID NO: 141.

The antibody according to the present disclosure may include not only the full-length antibody but also an antigen-binding fragment thereof. Specifically, the antigen-binding fragment may refer to a region except for Fc that functions to transmit the stimulus with an antigen to a cell, a complement, or the like. For example, the antigen-binding fragment of the antibody may include all of Fab, scFv, F(ab)2, and Fv, and may also include a 3rd generation antibody fragment, such as a single domain antibody and a minibody.

For example, the antibody or antigen-binding fragment thereof may include: a heavy chain variable region including a heavy chain CDR1 consisting of the amino acid sequence as set forth in SEQ ID NO: 89, a heavy chain CDR2 in which five or fewer amino acids are substituted in a polypeptide consisting of the amino acid sequence as set forth in SEQ ID NO: 90, and a heavy chain CDR3 consisting of the amino acid sequence as set forth in SEQ ID NO: 91; and a light chain variable region including a light chain CDR1 consisting of the amino acid sequence as set forth in SEQ ID NO: 92, a light chain CDR2, which is a polypeptide consisting of the amino acid sequence as set forth in SEQ ID NO: 93 or in which five or fewer amino acids are substituted in the polypeptide, and a light chain CDR3, which is a polypeptide consisting of the amino acid sequence as set forth in SEQ ID NO: 94 or in which two or fewer amino acids are substituted in the polypeptide.

The term "complementarity determining region (CDR)" refers to a hypervariable region that is in the heavy chain and light chain variable regions of an antibody and has a different amino acid sequence for each antibody, and means a region that actually binds to an antigen. In a three-dimensional conformation of an antibody, CDR has a loop shape on a surface of the antibody, wherein under the loop, a framework region (FR) may exist for structurally supporting CDR. There are three loop structures in each of the heavy chain and the light chain, and these six loop structures can be combined together to directly contact an antigen. The antigen-binding site having six loop structures, CDRs, may be, for convenience, heavy chain CDR1, heavy chain CDR2, heavy chain CDR3, light chain CDR1, light chain CDR2 or light chain CDR3, respectively.

In the antibody or antigen-binding fragment thereof according to the present disclosure, the heavy chain CDR2 in which five or fewer amino acids are substituted in a polypeptide consisting of the amino acid sequence as set forth in SEQ ID NO: 90 may have substitutions of five or fewer amino acids selected from the group consisting of amino acids at positions 2, 4 to 10, 12, 13, and 17 from the N-terminus of the polypeptide.

For example, the amino acid at positions 2, 4, 6, 7, 9, 12, or 13 from the N-terminus of the polypeptide consisting of the amino acid sequence as set forth in SEQ ID NO: 90 may be substituted with a neutral amino acid. Specifically, the neutral amino acid may include alanine, glycine, leucine, isoleucine, proline, valine, phenylalanine, tryptophan, tyrosine, serine, threonine, cysteine, methionine, asparagine, and glutamine. More specifically, isoleucine, which is the amino acid at position 2 from the N-terminus, may be substituted with leucine, valine, or threonine; tyrosine, which is the amino acid at position 4 from the N-terminus, may be substituted with phenylalanine or tryptophan; serine, which is the amino acid at position 6 from the N-terminus, may be substituted with glycine or asparagine; glycine, which is the amino acid at position 7 from the N-terminus, may be substituted with valine; isoleucine, which is the amino acid at position 9 from the N-terminus, may be substituted with proline, alanine, or valine; alanine, which is the amino acid at position 12 from the N-terminus, may be substituted with serine; and aspartic acid, which is the amino acid at position 13 from the N-terminus, may be substituted with asparagine or alanine. Meanwhile, the amino acid at position 10 from the N-terminus of the polypeptide consisting of the amino acid sequence as set forth in SEQ ID NO: 90 may be substituted with a basic amino acid, and specifically, the basic amino acid may include arginine, histidine, and lysine. More specifically, tyrosine, which is the amino acid at position 10 from the N-terminus, may be substituted with arginine. In addition, the amino acid at position 17 from the N-terminus of the polypeptide consisting of the amino acid sequence as set forth in SEQ ID NO: 90 may be substituted with an acidic amino acid, and specifically, the acidic amino acid may include aspartic acid and glutamic acid. More specifically, glycine, which is the amino acid at position 17 from the N-terminus, may be substituted with aspartic acid or glutamic acid. In addition, the amino acid at position 5 from the N-terminus of the polypeptide consisting of the amino acid sequence as set forth in SEQ ID NO: 90 may be substituted with a neutral or acidic amino acid, and specifically, the neutral or acidic amino acid may be as described above. More specifically, glycine, which is the amino acid at position 5 from the N-terminus, may be substituted with alanine, aspartic acid, serine, or proline. Furthermore, the amino acid at position 8 from the N-terminus of the polypeptide consisting of the amino acid sequence as set forth in SEQ ID NO: 90 may be substituted with a neutral, acidic, or basic amino acid, and the neutral, acidic, or basic amino acid may be as described above. More specifically, asparagine, which is the amino acid at position 8 from the N-terminus, may be substituted with aspartic acid, lysine, isoleucine, threonine, valine, glycine, or tyrosine.

In an embodiment of the present disclosure, the heavy chain CDR2 in which five or fewer amino acids are substituted in a polypeptide consisting of the amino acid sequence as set forth in SEQ ID NO: 90 may be a polypeptide consisting of any one of the amino acid sequences as set forth in SEQ ID NOS: 95 to 111.

In the antibody or antigen-binding fragment thereof according to the present disclosure, the light chain CDR2 in which five or fewer amino acids are substituted in a polypeptide consisting of the amino acid sequence as set forth in SEQ ID NO: 93 may have substitutions of four or fewer amino acids selected from the group consisting of amino acids at positions 3 to 7 from the N-terminus of the polypeptide.

For example, the amino acids at positions 3, 4, and 5 from the N-terminus of the polypeptide consisting of the amino acid sequence as set forth in SEQ ID NO: 93 may be substituted with a neutral amino acid, and the neutral amino acid may be as described above. More specifically, serine, which is the amino acid at position 3 from the N-terminus, may be substituted with isoleucine, leucine, methionine, glutamine, threonine, valine, tryptophan, or tyrosine; histidine, which is the amino acid at position 4 from the N-terminus, may be substituted with phenylalanine or tyrosine; and arginine, which is the amino acid at position 5 from the N-terminus, may be substituted with glutamine, proline, leucine, threonine, or serine. Meanwhile, the amino acid at position 6 from the N-terminus of the polypeptide consisting of the amino acid sequence as set forth in SEQ ID NO: 93 may be substituted with a basic or neutral amino acid, and the basic or neutral amino acid may be as described above. More specifically, proline, which is the amino acid at position 6 from the N-terminus, may be substituted with alanine, glutamine, arginine, serine, or histidine. Furthermore, the amino acid at position 7 from the N-terminus of the polypeptide consisting of the amino acid sequence as set forth in SEQ **ID NO:** 93 may be substituted with a neutral, acidic, or basic amino acid, and the neutral, acidic, or basic amino acid may be as described above. More specifically, serine, which is the amino acid at position 7 from the N-terminus, may be substituted with aspartic acid, tyrosine, glycine, asparagine, threonine, phenylalanine, tryptophan, or arginine.

In an embodiment of the present disclosure, the light chain CDR2 in which five or fewer amino acids are substituted in the polypeptide consisting of the amino acid sequence as set forth in SEQ ID **NO:** 93 may be a polypeptide consisting of any one of the amino acid sequences as set forth in SEQ ID NOS: 112 to 135.

In the antibody or antigen-binding fragment thereof according to the present disclosure, the light chain CDR2 in which two or fewer amino acids are substituted in the polypeptide consisting of the amino acid sequence as set forth in SEQ ID NO: 94 may have substitutions of two or fewer amino acids selected from the group consisting of amino acids at positions 6 and 8 from the N-terminus of the polypeptide.

For example, the amino acids at positions 6 and 8 from the N-terminus of the polypeptide consisting of the amino acid sequence as set forth in SEQ ID NO: 94 may be substituted with a neutral amino acid, and the neutral amino acid may be as described above. More specifically, serine residues, which are the amino acids at positions 6 and 8 from the N-terminus, may be substituted with tryptophan and glycine, respectively.

In an embodiment of the present disclosure, the light chain CDR3 in which two or fewer amino acids are substituted in a polypeptide consisting of the amino acid sequence as set forth in SEQ ID NO: 94 may be a polypeptide consisting of the amino acid sequence as set forth in SEQ ID NO: 136.

In addition, the antibody or antigen-binding fragment thereof according to the present disclosure may be modified as needed. Specifically, the antibody or antigen-binding fragment thereof may be modified by conjugation, glycosylation, labeling, or a combination thereof. Specifically, the antibody or antigen-binding fragment thereof may be modified with horseradish peroxidase (HRP), an alkaline phosphatase, hapten, biotin, streptavidin, a fluorescent substance, a radioactive substance, a quantum dot, polyethylene glycol (PEG), a histidine label, or the like. The antibody or antigen-binding fragment thereof may also be conjugated to another drug as needed.

The antibody or antigen-binding fragment thereof may be produced according to a monoclonal antibody production method known in the art, and the production method may be appropriately modified by a person skilled in the art. For example, the antibody may be produced by preparing hybridomas through the use of B lymphocytes obtained from an animal immunized with an antigen, or may be produced by phage display technology.

Furthermore, the present disclosure provides a nucleic acid encoding the antibody or antigen-binding fragment thereof.

An antibody or antigen-binding fragment thereof encoded by the nucleic acid according to the present disclosure may have the features as described above. As long as an amino acid sequence constituting the antibody or antigen-binding fragment thereof according to the present disclosure is known, a nucleic acid encoding the same would also be obvious to a person skilled in the art. The nucleic acid sequence may have at least one nucleotide addition, deletion, or substitution as long as the activity of the antibody or antigen-binding fragment thereof is retained.

Furthermore, the present disclosure provides an expression vector including the nucleic acid.

The nucleic acid included in the expression vector according to the present disclosure may encode the antibody or antigen-binding fragment thereof having the features as described above.

As used herein, the term "expression vector" is a means for expressing a target gene in a host cell, and may include all of a plasmid vector, a cosmid vector, a bacteriophage vector, a viral vector, and the like. The expression vector may include elements necessary for preparing the peptide from the nucleic acid included therein. Specifically, the expression vector may include a signal sequence, an origin of replication, a marker gene, a promoter, a transcription termination sequence, and the like. The nucleic acid encoding the antibody or antigen-binding fragment thereof according to the present disclosure may be operatively linked to the promoter.

As an example, an expression vector used in prokaryotic cells may include a promoter for transcription, a ribosome binding site for initiation of translation, and termination sequences for transcription and translation. The expression vector used in eukaryotic cells may include a promoter and polyadenylation sequence derived from a mammal or a mammalian virus.

All marker genes known in the art may be used as a marker gene included in the expression vector, and may be specifically an antibiotic resistant gene. Specifically, the antibiotic resistant gene may be a gene showing resistance to antibiotics including ampicillin, gentamicin, carbenicillin, chloramphenicol, streptomycin, kanamycin, neomycin, tetracycline, and the like.

In addition, the present disclosure provides a host cell including the nucleic acid or expression vector.

The nucleic acid or expression vector included in the host cell according to the present disclosure may have the features as described above. As an example, the nucleic acid can encode the antibody or antigen-binding fragment thereof that specifically binds to ASM protein according to the present disclosure, and the expression vector may include the nucleic acid as described above.

All types of cells known to be usable to produce antibodies or antigen-binding fragments thereof in the art may be used as a host cell. Specifically, the host cell may be a prokaryotic cell, yeast, or a eukaryotic cell. Examples of the prokaryotic cell may include *E. coli,* the genus Bacillus strains, the genus Streptomyces strain, the genus Pseudomonas strain, the genus Staphylococcus strain, and the like, and examples of the yeast may include Saccharomyces cerevisiae and the like. Example of the eukaryotic cell may include COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PERC6, SP2/0, NS-0, U20S, HT1080, and the like.

The host cell may be transfected with the nucleic acid or expression vector as described above according to a method known in the art. Specifically, the transfection may be performed by transient transfection, microinjection, transduction, cell infusion, calcium phosphate precipitation, liposome-mediated transfection, DEAE dextran-mediated transfection, polybrene-mediated transfection, electrical penetration, a gene gun, or the like. The methods may also be appropriately modified by a person skilled in the art.

Furthermore, the present disclosure provides a method for producing an antibody or antigen-binding fragment thereof that specifically binds to an ASM protein, the method including culturing the host cell to produce the antibody or antigen-binding fragment thereof.

An antibody or antigen-binding fragment thereof obtained by the production method according to the present disclosure may have the features as described above.

The culture may be performed using an appropriate culture medium depending on the type of host cell used for production, and appropriate supplements may be contained therein as necessary. The culture may be performed in an appropriate environment depending on the type of host cell.

The production method according to the present disclosure may further include recovering an antibody or antigen-binding fragment thereof produced in the host cell. The recovering may be performed according to a method known in the art, wherein the culture may be modified as needed by a person skilled in the art. For example, the recovery may be performed by removing impurities through centrifugation or ultrafiltration, and may be performed by further purifying the obtained resultant product by chromatography or the like. The chromatography may include affinity chromatography, cation chromatography, hydrophobic interaction chromatography, and the like.

Furthermore, the present disclosure provides a composition and kit for detecting an ASM protein, each including the antibody or antigen-binding fragment thereof.

The antibody or antigen-binding fragment thereof included in the composition and kit for detecting an ASM protein according to the present disclosure may have the features as described above.

Further, the composition may include a ligand that can specifically bind to the antibody or antigen-binding fragment thereof according to the present disclosure. The ligand may be a conjugate labeled with a detector, such as a chromogenic enzyme, a fluorescent substance, a radioisotope, or a colloid, or may be treated with streptavidin or avidin. The diagnostic composition of the present disclosure may further include, in addition to the reagents as described above, distilled water or a buffer to stably maintain the structures thereof.

The kit may be bound to a solid substrate to facilitate subsequent steps, such as washing of the antibody or antigen-binding fragment thereof contained therein, or separation of the complex. A synthetic resin, nitrocellulose, a glass substrate, a metal substrate, a glass fiber, a microsphere, or a micro-bead may be used as a solid substrate. In addition, polyester, polyvinyl chloride, polystyrene, polypropylene, PVDF or nylon may be used as a synthetic resin.

The kit may be manufactured by a conventional manufacturing method known to a person skilled in the art, and may further include a buffer, a stabilizer, an inactive protein, and the like.

Furthermore, the present disclosure provides a method for detecting an ASM protein, the method including reacting a sample with the antibody or antigen-binding fragment thereof.

An antibody or antigen-binding fragment thereof used in the ASM protein detection method according to the present disclosure may have the features as described above.

The sample may include all kinds of samples that are used to detect the ASM protein. A method for detecting a target protein by using an antibody or antigen-binding fragment thereof is well known in the art, and the method may be performed by a person skilled in the art through appropriate modification as needed.

Hereinafter, the present disclosure will be described in detail with reference to the following examples. However, the following exemplary embodiments are merely for illustrating the present disclosure, and are not intended to limit the scope of the present disclosure. Any embodiment that has substantially the same constitution as the technical idea described in the claims of the disclosure and achieves the same effects of action should be included in the technical scope of the present disclosure.

### Example 1: Production of Antibodies Specifically Binding to Acid Sphingomyelinase (ASM) Protein

Variants were produced from the antibody #9104, which is an antibody specifically binding to the human ASM protein (SEQ ID NO: 139).

First, VH and VL genes of the antibody #9104 were amplified by a conventional method using random primers for random mutagenesis in the heavy and light chain CDR sequences of the antibody #9104. The amplified VH and VL genes were linked to mouse heavy chain constant region 1 (CH1, SEQ ID NO: 142) and light chain constant region (CL, SEQ ID NO: 143) and inserted into the pComb3xss phagemid vector in an scFab form. A #9104 random mutagenesis library was constructed by using the vector, and screening was performed by a commonly used method using the library to select scFab specifically binding to the human ASM protein. Expression vectors were produced such that a human heavy chain constant region and a human light chain λ constant region consisting of the nucleotide sequences as set forth in SEQ ID NOS: 137 and 138, respectively, were linked to the carboxy termini of the heavy chain variable region and the light chain variable region of the selected scFab. As a result, the amino acid and nucleic acid sequences of the heavy chain variable region of the selected scFab are shown in Table 1, and the amino acid and nucleic acid sequences of the light chain variable region thereof are shown in Table 2.

**TABLE 1**

| Antibody # | Sequence | Sequence Number |
|---|---|---|
| #9104 | EVQLLESGGGLVQPGGSLRLSCAASGFTFSNYYMSWVRQAP GKGLEWVSGIYYGSGNIYYADSVKGRFTISRDNSKNTLYLQ MNSLRAEDTAVYYCARDTPGFDYWGQGTLVTVSS | SEQ ID NO: 1 |
| | GAGGTGCAGCTGTTGGAGTCTGGGGGAGGCTTGGTACAGCC TGGGGGGTCCCTGAGACTCTCCTGTGCAGCCTCCGGATTCA CCTTCTCCAACTACTACATGTCCTGGGTCCGACAGGCCCCT GGCAAAGGATTGGAATGGGTGTCCGGCATCTACTACGGCTC CGGCAACATCTACTATGCCGACTCCGTGAAGGGCAGATTCA CCATCTCTCGGGACAACTCCAAGAACACCCTGTACCTGCAG ATGAACTCCCTGAGAGCCGAGGACACCGCCGTGTACTACTG CGCTAGAGATACCCCTGGCTTCGACTATTGGGGCCAGGGCA CACTGGTCACCGTGAGCTCA | SEQ ID NO: 2 |
| #9104v-H02 | EVQLLESGGGLVQPGGSLRLSCAASGFTFSNYYMSWVRQAP GKGLEWVSGIYFGSGDIRYADSVKGRFTISRDNSKNTLYLQ MNSLRAEDTAVYYCARDTPGFDYWGQGTLVTVSS | SEQ ID NO: 3 |
| | GAGGTGCAGCTGTTGGAGTCTGGGGGAGGCTTGGTACAGCC TGGAGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGATCTATTTTGGTAG TGGTGATATAAGGTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 4 |
| #9104v-H05 | EVQLLESGGGLVQPGGSLRLSCAASGFTFSNYYMSWVRQAP GKGLEWVSGIYYGGVKIYYADSVKGRFTISRDNSKNTLYLQ MNSLRAEDTAVYYCARDTPGFDYWGQGTLVTVSS | SEQ ID NO: 5 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGATCTATTATGGTGG TGTTAAAATATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 6 |
| #9104v-H10 | EVQLLESGGGLVQPGGSLRLSCAASGFTFSNYYMSWVRQAP GKGLEWVSGIYYGNGIPYYADSVKDRFTISRDNSKNTLYLQ MNSLRAEDTAVYYCARDTPGFDYWGQGTLVTVSS | SEQ ID NO: 7 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGTATCTATTATGGTAA TGGTATTCCATATTACGCTGATTCTGTAAAAGATCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 8 |
| #9104v-H11 | EVQLLESGGGLVQPGGSLRLSCAASGFTFSNYYMSWVRQAP GKGLEWVSGIYYGSGIPYYADSVKERFTISRDNSKNTLYLQ MNSLRAEDTAVYYCARDTPGFDYWGQGTLVTVSS | SEQ ID NO: 9 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGATCTATTATGGTAG TGGTATTCCATATTACGCTGATTCTGTAAAAGAACGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 10 |
| #9104v-H13 | EVQLLESGGGLVQPGGSLRLSCAASGFTFSNYYMSWVRQAP GKGLEWVSGIYYGSGIPYYANSVKGRFTISRDNSKNTLYLQ MNSLRAEDTAVYYCARDTPGFDYWGQGTLVTVSS | SEQ ID NO: 11 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGATCTATTATGGTAG TGGTATACCCTATTACGCTAATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 12 |
| #9104v-H14 | EVQLLESGGGLVQPGGSLRLSCAASGFTFSNYYMSWVRQAP GKGLEWVSGIYYGSGKPYYADSVKGRFTISRDNSKNTLYLQ MNSLRAEDTAVYYCARDTPGFDYWGQGTLVTVSS | SEQ ID NO: 13 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGATCTATTATGGTAG TGGTAAACCATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 14 |
| #9104v-H17 | EVQLLESGGGLVQPGGSLRLSCAASGFTFSNYYMSWVRRAP GKGLEWVSGIYYGSGTPYYAASVKGRFTISRDNSKNTLYLQ MNSLRAEDTAVYYCARDTPGFDYWGQGTLVTVSS | SEQ ID NO: 15 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCGGGCTCCA GGGAAGGGGCTGGAGTGGGTCTCAGGGATCTATTATGGTAG TGGTACTCCATATTACGCTGCTTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 16 |
| #9104v-H18 | EVQLLESGGGLVQPGGSLRLSCAASGFTFSNYYMSWVRQAP GKGLEWVSGIYYGSGTPYYSDSVKGRFTISRDNSKNTLYLQ MNSLRAEDTAVYYCARDTPGFDYWGQGTLVTVSS | SEQ ID NO: 17 |
| | GAGGTGCAGCTGTTGGAGTCTGGGGGAGGCTTGGTACAGCC | SEQ ID |
| | TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGATCTACTATGGTAG TGGTACTCCATACTACTCTGACTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | NO: 18 |
| #9104v-H19 | EVQLLESGGGLVQPGGSLRLSCAASGFTFSNYYMSWVRQAP GKGLEWVSGIYYGSGVPYYADSVKGRFTISRDNSKNTLYLQ MNSLRAEDTAVYYCARDTPGFDYWGQGTLVTVSS | SEQ ID NO: 19 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGATCTACTATGGCAG TGGTGTTCCATATTACGCTGATTCTGTCAAAGGGCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 20 |
| #9104v-H22 | EVQLLESGGGLVQPGGSLRLSCAASGFTFSNYYMSWVRQAP GKGLEWVSGLYYGGGTPYYADSVKGRFTISRDNSKNTLYLQ MNSLRAEDTAVYYCARDTPGFDYWGQGTLVTVSS | SEQ ID NO: 21 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGCTCTATTATGGTGG TGGTACTCCATATTACGCTGATTCTGTAAAAGGTCGGTTCA CCATCTCCAGAGACAATTCCAAGAACACGCTGTATCTGCAA ATGAACAGCCTGAGAGCTGAGGACACGGCCGTGTATTACTG TGCGAGAGATACGCCTGGGTTCGACTACTGGGGCCAGGGTA CACTGGTCACCGTGAGCTCA | SEQ ID NO: 22 |
| #9104v-H24 | EVQLLESGGGLVQPGGSLRLSCAASGFTFSNYYMSWVRQAP GKGLEWVSGLYYGSGIPYYADSVKGRFTISRDNSKNTLYLQ MNSLRAEDTAVYYCARDTPGFDYWGQGTLVTVSS | SEQ ID NO: 23 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGTTGTATTATGGTAG TGGTATTCCATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG | SEQ ID NO: 24 |
| | TGCGAGAGATACGCCTGGGTTCGACTACTGGGGCCAGGGTA CACTGGTCACCGTGAGCTCA | |
| #9104v-H25 | EVQLLESGGGLVQPGGSLRLSCAASGFTFSNYYMSWVRQAP GKGLEWVSGTYYASGGIYYADSVKGRFTISRDNSKNTLYLQ MNSLRAEDTAVYYCARDTPGFDYWGQGTLVTVSS | SEQ ID NO: 25 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGACATATTATGCTAG TGGTGGTATATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 26 |
| #9104v-H26 | EVQLLESGGGLVQPGGSLRLSCAASGFTFSNYYMSWVRQAP GKGLEWVSGTYYASGNAYYADSVKGRFTISRDNSKNTLYLQ MNSLRAEDTAVYYCARDTPGFDYWGQGTLVTVSS | SEQ ID NO: 27 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGACCTATTATGCTAG TGGTAATGCATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 28 |
| #9104v-H27 | EVQLLESGGGLVQPGGSLRLSCAASGFTFSNYYMSWVRQAP GKGLEWVSGTYYDSGYVYYADSVKGRFTISRDNSKNTLYLQ MNSLRAEDTAVYYCARDTPGFDYWGQGTLVTVSS | SEQ ID NO: 29 |
| | GAGGTGCAGCTGTTGGAGTCTGGGGGAGGCTTGGTACAGCC TGGGGGGTCCCTGAGACTCTCCTGTGCAGCCTCCGGGTTCA CCTTTAGCAATTATTATATGAGCTGGGTCCGCCAGGCTCCA GGGAAGGGGCTGGAGTGGGTCTCAGGGACCTATTATGATAG TGGATATGTATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 30 |
| #9104v-H30 | EVQLLESGGGLVQPGGSLRLSCAASGFTFSNYYMSWVRQAP GKGLEWVSGTYYSSGGIYYADSVKGRFTISRDNSKNTLYLQ MNSLRAEDTAVYYCARDTPGFDYWGQGTLVTVSS | SEQ ID NO: 31 |
| | GAGGTGCAGCTGTTGGAGTCTGGGGGAGGCTTGGTACAGCC | SEQ ID |
| | TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGACCTATTATAGTAG TGGTGGTATATATTACGCTGATTCTGTCAAAGGCCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | NO: 32 |
| #9104v-H31 | EVQLLESGGGLVQPGGSLRLSCAASGFTFSNYYMSWVRQAP GKGLEWVSGVYYGSGTPYYADSVKGRFTISRDNSKNTLYLQ MNSLRAEDTAVYYCARDTPGFDYWGQGTLVTVSS | SEQ ID NO: 33 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGGTCTACTATGGTAG TGGTACTCCATATTACGCTGATTCTGTAAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 34 |
| #9104v-H33 | EVQLLESGGGLVQPGGSLRLSCAASGFTFSNYYMSWVRQAP GKGLEWVSGVYYPSGNPYYADSVKGRFTISRDNSKNTLYLQ MNSLRAEDTAVYYCARDTPGFDYWGQGTLVTVSS | SEQ ID NO: 35 |
| | GAGGTGCAGCTGTTGGAGTCTGGGGGAGGCTTGGTACAGCC TGGGGGGTCCCTGAGACTCTCCTGTGCAGCCTCCGGATTCA CCTTTAGCAATTATTATATGAGCTGGGTCCGCCAGGCTCCA GGGAAGGGGCTGGAGTGGGTCTCAGGGGTATATTATCCTAG TGGTAATCCATATTACGCTGATTCTGTTAAAGGTCGGTTCA CCATCTCCAGAGACAATTCCAAGAACACGCTGTATCTGCAA ATGAACAGCCTGAGAGCCGAGGACACGGCCGTGTATTACTG TGCGAGAGATACGCCTGGGTTCGACTACTGGGGCCAGGGTA CACTGGTCACCGTGAGCTCA | SEQ ID NO: 36 |

**TABLE 2**

| Antibody # | Sequence | SEQ ID NO |
|---|---|---|
| #9104 | QSVLTQPPSASGTPGQRVTISCTGSSSNIGNNAVNWYQQLPG TAPKLLIYYDSHRPSGVPDRFSGSKSGTSASLAISGLRSEDE ADYYCGAWDYSLSAYVFGGGTKLTVL | SEQ ID NO: 37 |
| | CAGTCTGTGCTGACTCAGCCACCCTCAGCTAGCGGCACACCT GGCCAGAGAGTGACCATCTCTTGTACCGGCTCCTCCTCCAAC | SEQ ID NO: 38 |
| | ATCGGCAACAACGCCGTGAACTGGTATCAGCAGCTGCCTGGC ACAGCCCCTAAACTGCTGATCTACTACGACTCCCACCGGCCT AGCGGCGTGCCCGATAGATTTTCCGGCTCTAAGTCCGGCACC TCTGCCAGCCTGGCTATCTCTGGACTGAGATCTGAGGACGAG GCCGACTACTACTGTGGCGCCTGGGACTACTCTCTGTCCGCC TATGTTTTTGGCGGAGGCACCAAGCTTACGGTCCTA | |
| #9104v-L11 | QSVLTQPPSASGTPGQRVTISCTGSSSNIGNNAVNWYQQLPG TAPKLLIYYDIFQPSGVPDRFSGSKSGTSASLAISGLRSEDE ADYYCGAWDYSLSAYVFGGGTKLTVL | SEQ ID NO: 39 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATATTTTTCAGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 40 |
| #9104v-L12 | QSVLTQPPSASGTPGQRVTISCTGSSSNIGNNAVNWYQQLPG TAPKLLIYYDIFRPDGVPDRFSGSKSGTSASLAISGLRSEDE ADYYCGAWDYSLSAYVFGGGTKLTVL | SEQ ID NO: 41 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATATTTTTCGGCCA GACGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 42 |
| #9104v-L15 | QSVLTQPPSASGTPGQRVTISCTGSSSNIGNNAVNWYQQLPG TAPKLLIYYDIHRPYGVPDRFSGSKSGTSASLAISGLRSEDE ADYYCGAWDYSLSAYVFGGGTKLTVL | SEQ ID NO: 43 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATATTCATCGGCCA TACGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 44 |
| #9104v-L19 | QSVLTQPPSASGTPGQRVTISCTGSSSNIGNNAVNWYQQLPG TAPKLLIYYDLFRPSGVPDRFSGSKSGTSASLAISGLRSEDE ADYYCGAWDYSLSAYVFGGGTKLTVL | SEQ ID NO: 45 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATCTTTTTAGGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 406 |
| #9104v-L20 | QSVLTQPPSASGTPGQRVTISCTGSSSNIGNNAVNWYQQLPG TAPKLLIYYDMFRPGGVPDRFSGSKSGTSASLAISGLRSEDE ADYYCGAWDYSLSAYVFGGGTKLTVL | SEQ ID NO: 47 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTACGATATGTTCCGGCCA GGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 48 |
| #9104v-L21 | QSVLTQPPSASGTPGQRVTISCTGSSSNIGNNAVNWYQQLPG TAPKLLIYYDMFRPNGVPDRFSGSKSGTSASLAISGLRSEDE ADYYCGAWDYSLSAYVFGGGTKLTVL | SEQ ID NO: 49 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATATGTTCCGGCCA AACGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 50 |
| #9104v-L23 | QSVLTQPPSASGTPGQRVTISCTGSSSNIGNNAVNWYQQLPG TAPKLLIYYDQFRPSGVPDRFSGSKSGTSASLAISGLRSEDE ADYYCGAWDYSLSAYVFGGGTKLTVL | SEQ ID NO: 51 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATCAATTTCGGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 52 |
| #9104v-L25 | QSVLTQPPSASGTPGQRVTISCTGSSSNIGNNAVNWYQQLPG | SEQ ID |
| | TAPKLLIYYDSFPASGVPDRFSGSKSGTSASLAISGLRSEDE ADYYCGAWDYSLSAYVFGGGTKLTVL | NO: 53 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATAGTTTTCCGGCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 54 |
| #9104v-L27 | QSVLTQPPSASGTPGQRVTISCTGSSSNIGNNAVNWYQQLPG TAPKLLIYYDSFRQSGVPDRFSGSKSGTSASLAISGLRSEDE ADYYCGAWDYSLSAYVFGGGTKLTVL | SEQ ID NO: 55 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATAGTTTTCGGCAA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 56 |
| #9104v-L28 | QSVLTQPPSASGTPGQRVTISCTGSSSNIGNNAVNWYQQLPG TAPKLLIYYDSFRRSGVPDRFSGSKSGTSASLAISGLRSEDE ADYYCGAWDYSLSAYVFGGGTKLTVL | SEQ ID NO: 57 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATAGTTTTCGGCGA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 58 |
| #9104v-L29 | QSVLTQPPSASGTPGQRVTISCTGSSSNIGNNAVNWYQQLPG TAPKLLIYYDSFRSTGVPDRFSGSKSGTSASLAISGLRSEDE ADYYCGAWDYSLSAYVFGGGTKLTVL | SEQ ID NO: 59 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTACGATAGTTTTCGGTCA ACCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT | SEQ ID NO: 60 |
| | TATGTCTTCGGCGGAGGCACCAAGCTTACGGTCCTA | |
| #9104v-L32 | QSVLTQPPSASGTPGQRVTISCTGSSSNIGNNAVNWYQQLPG TAPKLLIYYDSYLPSGVPDRFSGSKSGTSASLAISGLRSEDE ADYYCGAWDYSLSAYVFGGGTKLTVL | SEQ ID NO: 61 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATAGTTATCTGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 62 |
| #9104v-L33 | QSVLTQPPSASGTPGQRVTISCTGSSSNIGNNAVNWYQQLPG TAPKLLIYYDSYPPYGVPDRFSGSKSGTSASLAISGLRSEDE ADYYCGAWDYSLSAYVFGGGTKLTVL | SEQ ID NO: 63 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATAGTTATCCGCCA TACGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 64 |
| #9104v-L34 | QSVLTQPPSASGTPGQRVTISCTGSSSNIGNNAVNWYQQLPG TAPKLLIYYDSYQASGVPDRFSGSKSGTSASLAISGLRSEDE ADYYCGAWDYSLSAYVFGGGTKLTVL | SEQ ID NO: 65 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATAGTTATCAGGCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 66 |
| #9104v-L37 | QSVLTQPPSASGTPGQRVTISCTGSSSNIGNNAVNWYQQLPG TAPKLLIYYDSYRASGVPDRFSGSKSGTSASLAISGLRSEDE ADYYCGAWDYSLSAYVFGGGTKLTVL | SEQ ID NO: 67 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATAGTTATCGGGCA AGCGGGGTCCCTGACCGATTCTCTGGCTCCAAGTCTGGCACC | SEQ ID NO: 68 |
| | TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | |
| #9104v-L38 | QSVLTQPPSASGTPGQRVTISCTGSSSNIGNNAVNWYQQLPG TAPKLLIYYDSYRPFGVPDRFSGSKSGTSASLAISGLRSEDE ADYYCGAWDYSLSAYVFGGGTKLTVL | SEQ ID NO: 69 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATAGTTATCGGCCA TTCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 70 |
| #9104v-L39 | QSVLTQPPSASGTPGQRVTISCTGSSSNIGNNAVNWYQQLPG TAPKLLIYYDSYRPGGVPDRFSGSKSGTSASLAISGLRSEDE ADYYCGAWDYSLSAYVFGGGTKLTVL | SEQ ID NO: 71 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGACAGTTATCGGCCA GGGGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 72 |
| #9104v-L46 | QSVLTQPPSASGTPGQRVTISCTGSSSNIGNNAVNWYQQLPG TAPKLLIYYDSYRSWGVPDRFSGSKSGTSASLAISGLRSEDE ADYYCGAWDYSLSAYVFGGGTKLTVL | SEQ ID NO: 73 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATAGTTATCGGTCA TGGGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 74 |
| #9104v-L47 | QSVLTQPPSASGTPGQRVTISCTGSSSNIGNNAVNWYQQLPG TAPKLLIYYDSYTPSGVPDRFSGSKSGTSASLAISGLRSEDE ADYYCGAWDYSLSAYVFGGGTKLTVL | SEQ ID NO: 75 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA | SEQ ID NO: 76 |
| | ACGGCCCCCAAACTCCTCATCTATTATGATAGTTATACGCCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | |
| #9104v-L49 | QSVLTQPPSASGTPGQRVTISCTGSSSNIGNNAVNWYQQLPG TAPKLLIYYDTFRPTGVPDRFSGSKSGTSASLAISGLRSEDE ADYYCGAWDYSLSAYVFGGGTKLTVL | SEQ ID NO: 77 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATACTTTCCGGCCA ACCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 78 |
| #9104v-L52 | QSVLTQPPSASGTPGQRVTISCTGSSSNIGNNAVNWYQQLPG TAPKLLIYYDVHRPRGVPDRFSGSKSGTSASLAISGLRSEDE ADYYCGAWDYSLSAYVFGGGTKLTVL | SEQ ID NO: 79 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATGTTCATCGGCCC AGGGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 80 |
| #9104v-L55 | QSVLTQPPSASGTPGQRVTISCTGSSSNIGNNAVNWYQQLPG TAPKLLIYYDWYRPTGVPDRFSGSKSGTSASLAISGLRSEDE ADYYCGAWDYSLSAYVFGGGTKLTVL | SEQ ID NO: 81 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATTGGTATCGGCCA ACCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 82 |
| #9104v-L63 | QSVLTQPPSASGTPGQRVTISCTGSSSNIGNNAVNWYQQLPG TAPKLLIYYDSHRPSGVPDRFSGSKSGTSASLAISGLRSEDE ADYYCGAWDYWLGAYVFGGGTKLTVL | SEQ ID NO: 83 |
| | CAGTCTGTGCTGACTCAGCCACCCTCAGCTAGCGGGACCCCC | SEQ ID |
| | GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATAGTCATCGGCCA AGCGGGGTCCCTGACCGATTCTCTGGCTCCAAGTCTGGCACC TCAGCCTCCCTGGCCATTAGTGGGCTCCGGTCCGAGGATGAG GCTGATTATTACTGTGGTGCTTGGGATTATTGGCTGGGTGCT TATGTCTTCGGCGGAGGCACCAAGCTTACGGTCCTA | NO: 84 |
| #9104v-L64 | QSVLTQPPSASGTPGQRVTISCTGSSSNIGNNAVNWYQQLPG TAPKLLIYYDYFSHSGVPDRFSGSKSGTSASLAISGLRSEDE ADYYCGAWDYSLSAYVFGGGTKLTVL | SEQ ID NO: 85 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATTATTTTAGCCAT AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 86 |
| #9104v-L65 | QSVLTQPPSASGTPGQRVTISCTGSSSNIGNNAVNWYQQLPG TAPKLLIYYDYYQSSGVPDRFSGSKSGTSASLAISGLRSEDE ADYYCGAWDYSLSAYVFGGGTKLTVL | SEQ ID NO: 87 |
| | CAGTCTGTGCTGACTCAGCCACCCTCAGCTAGCGGGACCCCC GGGCAGAGGGTCACCATCTCTTGTACTGGCTCTTCATCTAAT ATTGGCAATAATGCTGTCAACTGGTACCAGCAGCTCCCAGGA ACGGCCCCCAAACTCCTCATCTATTATGATTATTATCAGTCA AGCGGGGTCCCTGACCGATTCTCTGGCTCCAAGTCTGGCACC TCAGCCTCCCTGGCCATCAGTGGGCTCCGGTCCGAGGATGAG GCTGATTATTACTGTGGTGCTTGGGATTATAGCCTGAGTGCT TATGTCTTCGGCGGAGGCACCAAGCTTACGGTCCTA | SEQ ID NO: 88 |

The expression vector employed a mammalian expression vector. The produced expression vectors were transformed into the ExpiCHO cell line to produce full-length antibodies binding to human ASM protein.

### Example 2: Determination of Complementarity Determining Regions (CDRs)

The complementarity determining regions in the produced scFv fragments were identified by a conventional method, and as a result, the CDR sequences of the heavy chain variable regions are shown in Table 3 and the CDR sequences of the light chain variable regions are shown in Table 4.

**TABLE 3**

| Antibody # | CDR | Sequence | SEQ ID NO |
|---|---|---|---|
| #9104 | CDR1 | NYYMS | SEQ ID NO: 89 |
| | CDR2 | GIYYGSGNIYYADSVKG | SEQ ID NO: 90 |
| | CDR3 | DTPGFDY | SEQ ID NO: 91 |
| #9104v-H02 | CDR1 | NYYMS | SEQ ID NO: 89 |
| | CDR2 | GIYFGSGDIRYADSVKG | SEQ ID NO: 95 |
| | CDR3 | DTPGFDY | SEQ ID NO: 91 |
| #9104v-H05 | CDR1 | NYYMS | SEQ ID NO: 89 |
| | CDR2 | GIYYGGVKIYYADSVKG | SEQ ID NO: 96 |
| | CDR3 | DTPGFDY | SEQ ID NO: 91 |
| #9104v-H10 | CDR1 | NYYMS | SEQ ID NO: 89 |
| | CDR2 | GIYYGNGIPYYADSVKD | SEQ ID NO: 97 |
| | CDR3 | DTPGFDY | SEQ ID NO: 91 |
| #9104v-H11 | CDR1 | NYYMS | SEQ ID NO: 89 |
| | CDR2 | GIYYGSGIPYYADSVKE | SEQ ID NO: 98 |
| | CDR3 | DTPGFDY | SEQ ID NO: 91 |
| #9104v-H13 | CDR1 | NYYMS | SEQ ID NO: 89 |
| | CDR2 | GIYYGSGIPYYANSVKG | SEQ ID NO: 99 |
| | CDR3 | DTPGFDY | SEQ ID NO: 91 |
| #9104v-H14 | CDR1 | NYYMS | SEQ ID NO: 89 |
| | CDR2 | GIYYGSGKPYYADSVKG | SEQ ID NO: 100 |
| | CDR3 | DTPGFDY | SEQ ID NO: 91 |
| #9104v-H17 | CDR1 | NYYMS | SEQ ID NO: 89 |
| | CDR2 | GIYYGSGTPYYAASVKG | SEQ ID NO: 101 |
| | CDR3 | DTPGFDY | SEQ ID NO: 91 |
| #9104v-H18 | CDR1 | NYYMS | SEQ ID NO: 89 |
| | CDR2 | GIYYGSGTPYYSDSVKG | SEQ ID NO: 102 |
| | CDR3 | DTPGFDY | SEQ ID NO: 91 |
| #9104v-H19 | CDR1 | NYYMS | SEQ ID NO: 89 |
| | CDR2 | GIYYGSGVPYYADSVKG | SEQ ID NO: 103 |
| | CDR3 | DTPGFDY | SEQ ID NO: 91 |
| #9104v-H22 | CDR1 | NYYMS | SEQ ID NO: 89 |
| | CDR2 | GLYYGGGTPYYADSVKG | SEQ ID NO: 104 |
| | CDR3 | DTPGFDY | SEQ ID NO: 91 |
| #9104v-H24 | CDR1 | NYYMS | SEQ ID NO: 89 |
| | CDR2 | GLYYGSGIPYYADSVKG | SEQ ID NO: 105 |
| | CDR3 | DTPGFDY | SEQ ID NO: 91 |
| #9104v-H25 | CDR1 | NYYMS | SEQ ID NO: 89 |
| | CDR2 | GTYYASGGIYYADSVKG | SEQ ID NO: 106 |
| | CDR3 | DTPGFDY | SEQ ID NO: 91 |
| #9104v-H26 | CDR1 | NYYMS | SEQ ID NO: 89 |
| | CDR2 | GTYYASGNAYYADSVKG | SEQ ID NO: 107 |
| | CDR3 | DTPGFDY | SEQ ID NO: 91 |
| #9104v-H27 | CDR1 | NYYMS | SEQ ID NO: 89 |
| | CDR2 | GTYYDSGYVYYADSVKG | SEQ ID NO: 108 |
| | CDR3 | DTPGFDY | SEQ ID NO: 91 |
| #9104v-H30 | CDR1 | NYYMS | SEQ ID NO: 89 |
| | CDR2 | GTYYSSGGIYYADSVKG | SEQ ID NO: 109 |
| | CDR3 | DTPGFDY | SEQ ID NO: 91 |
| #9104v-H31 | CDR1 | NYYMS | SEQ ID NO: 89 |
| | CDR2 | GVYYGSGTPYYADSVKG | SEQ ID NO: 110 |
| | CDR3 | DTPGFDY | SEQ ID NO: 91 |
| #9104v-H33 | CDR1 | NYYMS | SEQ ID NO: 89 |
| | CDR2 | GVYYPSGNPYYADSVKG | SEQ ID NO: 111 |
| | CDR3 | DTPGFDY | SEQ ID NO: 91 |

**TABLE 4**

| Antibody # | CDR | Sequence | SEQ ID NO |
|---|---|---|---|
| #9104 | CDR1 | TGSSSNIGNNAVN | SEQ ID NO: 92 |
| | CDR2 | YDSHRPS | SEQ ID NO: 93 |
| | CDR3 | GAWDYSLSAYV | SEQ ID NO: 94 |
| #9104v-L11 | CDR1 | TGSSSNIGNNAVN | SEQ ID NO: 92 |
| | CDR2 | YDIFQPS | SEQ ID NO: 112 |
| | CDR3 | GAWDYSLSAYV | SEQ ID NO: 94 |
| #9104v-L12 | CDR1 | TGSSSNIGNNAVN | SEQ ID NO: 92 |
| | CDR2 | YDIFRPD | SEQ ID NO: 113 |
| | CDR3 | GAWDYSLSAYV | SEQ ID NO: 94 |
| #9104v-L15 | CDR1 | TGSSSNIGNNAVN | SEQ ID NO: 92 |
| | CDR2 | YDIHRPY | SEQ ID NO: 114 |
| | CDR3 | GAWDYSLSAYV | SEQ ID NO: 94 |
| #9104v-L19 | CDR1 | TGSSSNIGNNAVN | SEQ ID NO: 92 |
| | CDR2 | YDLFRPS | SEQ ID NO: 115 |
| | CDR3 | GAWDYSLSAYV | SEQ ID NO: 94 |
| #9104v-L20 | CDR1 | TGSSSNIGNNAVN | SEQ ID NO: 92 |
| | CDR2 | YDMFRPG | SEQ ID NO: 116 |
| | CDR3 | GAWDYSLSAYV | SEQ ID NO: 94 |
| #9104v-L21 | CDR1 | TGSSSNIGNNAVN | SEQ ID NO: 92 |
| | CDR2 | YDMFRPN | SEQ ID NO: 117 |
| | CDR3 | GAWDYSLSAYV | SEQ ID NO: 94 |
| #9104v-L23 | CDR1 | TGSSSNIGNNAVN | SEQ ID NO: 92 |
| | CDR2 | YDQFRPS | SEQ ID NO: 118 |
| | CDR3 | GAWDYSLSAYV | SEQ ID NO: 94 |
| #9104v-L25 | CDR1 | TGSSSNIGNNAVN | SEQ ID NO: 92 |
| | CDR2 | YDSFPAS | SEQ ID NO: 119 |
| | CDR3 | GAWDYSLSAYV | SEQ ID NO: 94 |
| #9104v-L27 | CDR1 | TGSSSNIGNNAVN | SEQ ID NO: 92 |
| | CDR2 | YDSFRQS | SEQ ID NO: 120 |
| | CDR3 | GAWDYSLSAYV | SEQ ID NO: 94 |
| #9104v-L28 | CDR1 | TGSSSNIGNNAVN | SEQ ID NO: 92 |
| | CDR2 | YDSFRRS | SEQ ID NO: 121 |
| | CDR3 | GAWDYSLSAYV | SEQ ID NO: 94 |
| #9104v-L29 | CDR1 | TGSSSNIGNNAVN | SEQ ID NO: 92 |
| | CDR2 | YDSFRST | SEQ ID NO: 122 |
| | CDR3 | GAWDYSLSAYV | SEQ ID NO: 94 |
| #9104v-L32 | CDR1 | TGSSSNIGNNAVN | SEQ ID NO: 92 |
| | CDR2 | YDSYLPS | SEQ ID NO: 123 |
| | CDR3 | GAWDYSLSAYV | SEQ ID NO: 94 |
| #9104v-L33 | CDR1 | TGSSSNIGNNAVN | SEQ ID NO: 92 |
| | CDR2 | YDSYPPY | SEQ ID NO: 124 |
| | CDR3 | GAWDYSLSAYV | SEQ ID NO: 94 |
| #9104v-L34 | CDR1 | TGSSSNIGNNAVN | SEQ ID NO: 92 |
| | CDR2 | YDSYQAS | SEQ ID NO: 125 |
| | CDR3 | GAWDYSLSAYV | SEQ ID NO: 94 |
| #9104v-L37 | CDR1 | TGSSSNIGNNAVN | SEQ ID NO: 92 |
| | CDR2 | YDSYRAS | SEQ ID NO: 126 |
| | CDR3 | GAWDYSLSAYV | SEQ ID NO: 94 |
| #9104v-L38 | CDR1 | TGSSSNIGNNAVN | SEQ ID NO: 92 |
| | CDR2 | YDSYRPF | SEQ ID NO: 127 |
| | CDR3 | GAWDYSLSAYV | SEQ ID NO: 94 |
| #9104v-L39 | CDR1 | TGSSSNIGNNAVN | SEQ ID NO: 92 |
| | CDR2 | YDSYRPG | SEQ ID NO: 128 |
| | CDR3 | GAWDYSLSAYV | SEQ ID NO: 94 |
| #9104v-L46 | CDR1 | TGSSSNIGNNAVN | SEQ ID NO: 92 |
| | CDR2 | YDSYRSW | SEQ ID NO: 129 |
| | CDR3 | GAWDYSLSAYV | SEQ ID NO: 94 |
| #9104v-L47 | CDR1 | TGSSSNIGNNAVN | SEQ ID NO: 92 |
| | CDR2 | YDSYTPS | SEQ ID NO: 130 |
| | CDR3 | GAWDYSLSAYV | SEQ ID NO: 94 |
| #9104v-L49 | CDR1 | TGSSSNIGNNAVN | SEQ ID NO: 92 |
| | CDR2 | YDTFRPT | SEQ ID NO: 131 |
| | CDR3 | GAWDYSLSAYV | SEQ ID NO: 94 |
| #9104v-L52 | CDR1 | TGSSSNIGNNAVN | SEQ ID NO: 92 |
| | CDR2 | YDVHRPR | SEQ ID NO: 132 |
| | CDR3 | GAWDYSLSAYV | SEQ ID NO: 94 |
| #9104v-L55 | CDR1 | TGSSSNIGNNAVN | SEQ ID NO: 92 |
| | CDR2 | YDWYRPT | SEQ ID NO: 133 |
| | CDR3 | GAWDYSLSAYV | SEQ ID NO: 94 |
| #9104v-L63 | CDR1 | TGSSSNIGNNAVN | SEQ ID NO: 92 |
| | CDR2 | YDSHRPS | SEQ ID NO: 93 |
| | CDR3 | GAWDYWLGAYV | SEQ ID NO: 136 |
| #9104v-L64 | CDR1 | TGSSSNIGNNAVN | SEQ ID NO: 92 |
| | CDR2 | YDYFSHS | SEQ ID NO: 134 |
| | CDR3 | GAWDYSLSAYV | SEQ ID NO: 94 |
| #9104v-L65 | CDR1 | TGSSSNIGNNAVN | SEQ ID NO: 92 |
| | CDR2 | YDYYQSS | SEQ ID NO: 135 |
| | CDR3 | GAWDYSLSAYV | SEQ ID NO: 94 |

As shown in Tables 3 and 4, CDR1 and CDR3 of the heavy chain variable region and CDR1 of the light chain variable region had the same sequences as in the antibody #9104, while CDR2 of the heavy chain variable region and CDR2, and CDR3 of the light chain variable region had sequences in which some constituent amino acids thereof are substituted.

### Experimental Example 1: Identification of Binding Affinity to ASM Protein

The binding affinity, to the ASM protein, of the produced antibodies specifically binding to the ASM protein and the interactive dynamics therebetween were measured using an Octet^{®} QK384 system (Pall Life Sciences).

First, an antibody specifically binding to the ASM protein produced in Example 1 was captured using an antihuman IgG Fc capture (AHC) biosensor, and 1.25, 2.5, 5, 10, or 20 nM recombinant human ASM protein solution was added thereto. After the addition of the human ASM protein solution, the association phase of the reaction product was observed for up to about 1,200 seconds. Then, 1X kinetics buffer (ForteBio) was added, and the dissociation phase of the reaction product was observed for about 1,500 seconds. The association constant (Kₐ), dissociation constant (K_{d}), and equilibrium dissociation constant (K_{D}) for each antibody were determined using Octet^{®} analysis software (Pall Life^{®} Sciences). As a result, the binding affinities, to the human ASM protein, of the antibodies with mutations in the heavy chain CDR regions of the antibody #9104 as depicted in Table 1 are shown in Table 5, and the binding affinities, to the human ASM protein, of the antibodies with mutations of the light chain CDR regions in the antibody #9104 as depicted in Table 2 are shown in Table 6.

**TABLE 5**

| Antibody # | K_{D} (M) | Kₐ (1/Ms) | K_{d} (1/s) |
|---|---|---|---|
| #9104 | 2.25E-09 | 3.89E+05 | 8.73E-04 |
| #9104v-H02 | 1.20E-09 | 2.78E+05 | 3.33E-04 |
| #9104v-H05 | 1.03E-09 | 1.71E+05 | 1.77E-04 |
| #9104v-H10 | 6.51E-10 | 2.37E+05 | 1.54E-04 |
| #9104v-H11 | 7.81E-10 | 2.50E+05 | 1.95E-04 |
| #9104v-H13 | 8.03E-10 | 2.31E+05 | 1.85E-04 |
| #9104v-H14 | 8.57E-10 | 3.28E+05 | 2.81E-04 |
| #9104v-H17 | 7.19E-10 | 3.38E+05 | 2.43E-04 |
| #9104v-H18 | 2.69E-09 | 9.79E+04 | 2.64E-04 |
| #9104v-H19 | 6.92E-10 | 2.66E+05 | 1.84E-04 |
| #9104v-H22 | 7.98E-10 | 1.97E+05 | 1.57E-04 |
| #9104v-H24 | 1.01E-09 | 2.04E+05 | 2.06E-04 |
| #9104v-H25 | 9.17E-10 | 2.07E+05 | 1.90E-04 |
| #9104v-H26 | 1.74E-09 | 1.75E+05 | 3.05E-04 |
| #9104v-H27 | 5.98E-10 | 2.76E+05 | 1.65E-04 |
| #9104v-H30 | 7.62E-10 | 1.99E+05 | 1.51E-04 |
| #9104v-H31 | 5.32E-10 | 2.66E+05 | 1.41E-04 |
| #9104v-H33 | 5.17E-10 | 2.08E+05 | 1.08E-04 |

**TABLE 6**

| Antibody # | K_{D} (M) | Kₐ (1/Ms) | K_{d} (1/s) |
|---|---|---|---|
| #9104 | 2.25E-09 | 3.89E+05 | 8.73E-04 |
| #9104v-L11 | 7.68E-10 | 2.59E+05 | 1.99E-04 |
| #9104v-L12 | 7.18E-10 | 2.21E+05 | 1.59E-04 |
| #9104v-L15 | 9.92E-10 | 2.75E+05 | 2.73E-04 |
| #9104v-L19 | 1.03E-09 | 2.31E+05 | 2.38E-04 |
| #9104v-L20 | 6.58E-10 | 2.47E+05 | 1.62E-04 |
| #9104v-L21 | 5.56E-10 | 2.43E+05 | 1.35E-04 |
| #9104v-L23 | 8.17E-10 | 2.26E+05 | 1.85E-04 |
| #9104v-L25 | 8.82E-10 | 2.94E+05 | 2.59E-04 |
| #9104v-L27 | 1.02E-09 | 3.48E+05 | 3.55E-04 |
| #9104v-L28 | 1.05E-09 | 2.41E+05 | 2.54E-04 |
| #9104v-L29 | 1.07E-09 | 2.72E+05 | 2.92E-04 |
| #9104v-L32 | 1.37E-09 | 3.21E+05 | 4.39E-04 |
| #9104v-L33 | 1.27E-09 | 2.27E+05 | 2.89E-04 |
| #9104v-L34 | 1.61E-09 | 3.06E+05 | 4.93E-04 |
| #9104v-L37 | 1.27E-09 | 2.59E+05 | 3.27E-04 |
| #9104v-L38 | 1.80E-09 | 1.91E+05 | 3.43E-04 |
| #9104v-L39 | 1.52E-09 | 2.91E+05 | 4.43E-04 |
| #9104v-L46 | 8.80E-10 | 2.93E+05 | 2.58E-04 |
| #9104v-L47 | 1.41E-09 | 2.94E+05 | 4.15E-04 |
| #9104v-L49 | 7.85E-10 | 2.86E+05 | 2.25E-04 |
| #9104v-L52 | 9.82E-10 | 2.69E+05 | 2.64E-04 |
| #9104v-L55 | 8.79E-10 | 1.93E+05 | 1.70E-04 |
| #9104v-L63 | 1.41E-09 | 1.81E+05 | 2.55E-04 |
| #9104v-L64 | 5.05E-10 | 3.83E+05 | 1.93E-04 |
| #9104v-L65 | 5.32E-10 | 3.60E+05 | 1.92E-04 |

As shown in Tables 5 and 6, the antibodies produced in Example 1 bound to the human ASM protein with a binding affinity of 10⁻¹⁰ to 10⁻⁹ M.

### Experimental Example 2: Combination of Heavy Chain and Light Chain Variable Regions

The selected 8 types of heavy chain variable regions and 5 types of light chain variable regions of the antibody #9104 were combined to produce 40 types of antibodies (Table 7).

**TABLE 7**

| Antibody # | Heavy chain variable region | Light chain variable region |
|---|---|---|
| #9104a-A1 | #9104v-H10 | #9104v-L20 |
| #9104a-A2 | #9104v-H11 | |
| #9104a-A3 | #9104v-H13 | |
| #9104a-A4 | #9104v-H17 | |
| #9104a-A5 | #9104v-H19 | |
| #9104a-A6 | #9104v-H27 | |
| #9104a-A7 | #9104v-H31 | |
| #9104a-A8 | #9104v-H33 | |
| #9104a-B1 | #9104v-H10 | #9104v-L21 |
| #9104a-B2 | #9104v-H11 | |
| #9104a-B3 | #9104v-H13 | |
| #9104a-B4 | #9104v-H17 | |
| #9104a-B5 | #9104v-H19 | |
| #9104a-B6 | #9104v-H27 | |
| #9104a-B7 | #9104v-H31 | |
| #9104a-B8 | #9104v-H33 | |
| #9104a-C1 | #9104v-H10 | #9104v-L23 |
| #9104a-C2 | #9104v-H11 | |
| #9104a-C3 | #9104v-H13 | |
| #9104a-C4 | #9104v-H17 | |
| #9104a-C5 | #9104v-H19 | |
| #9104a-C6 | #9104v-H27 | |
| #9104a-C7 | #9104v-H31 | |
| #9104a-C8 | #9104v-H33 | |
| #9104a-D1 | #9104v-H10 | #9104v-L64 |
| #9104a-D2 | #9104v-H11 | |
| #9104a-D3 | #9104v-H13 | |
| #9104a-D4 | #9104v-H17 | |
| #9104a-D5 | #9104v-H19 | |
| #9104a-D6 | #9104v-H27 | |
| #9104a-D7 | #9104v-H31 | |
| #9104a-D8 | #9104v-H33 | |
| #9104a-E1 | #9104v-H10 | #9104v-L65 |
| #9104a-E2 | #9104v-H11 | |
| #9104a-E3 | #9104v-H13 | |
| #9104a-E4 | #9104v-H17 | |
| #9104a-E5 | #9104v-H19 | |
| #9104a-E6 | #9104v-H27 | |
| #9104a-E7 | #9104v-H31 | |
| #9104a-E8 | #9104v-H33 | |

After the small-scale expression in the ExpiCHO cell line as described above, the binding affinity to the human ASM protein was investigated as described in Experimental Example 1. Specifically, the antibody produced by a combination of #9104v-H33 heavy chain variable region and #9104v-L23 light chain variable region was not expressed and thus excluded in the analysis of binding affinity to ASM protein. As a result, the binding affinities of the antibodies to human ASM protein are shown in Table 8.

**TABLE 8**

| Antibody # | K_{D} (M) | Kₐ (1/Ms) | K_{d} (1/s) |
|---|---|---|---|
| #9104 | 2.19E-09 | 3.91E+05 | 8.55E-04 |
| #9104a-A1 | 5.18E-11 | 2.20E+05 | 1.14E-05 |
| #9104a-A2 | 9.61E-11 | 2.06E+05 | 1.98E-05 |
| #9104a-A3 | 2.77E-11 | 1.98E+05 | 5.48E-06 |
| #9104a-A4 | 1.62E-10 | 2.19E+05 | 3.54E-05 |
| #9104a-A5 | 1.60E-10 | 2.46E+05 | 3.93E-05 |
| #9104a-A6 | 7.68E-10 | 9.06E+04 | 6.96E-05 |
| #9104a-A7 | 3.54E-11 | 2.30E+05 | 8.13E-06 |
| #9104a-A8 | 2.37E-10 | 1.91E+05 | 4.51E-05 |
| #9104a-B1 | 1.37E-10 | 2.53E+05 | 3.45E-05 |
| #9104a-B2 | 1.14E-10 | 2.66E+05 | 3.03E-05 |
| #9104a-B3 | 3.51E-11 | 2.34E+05 | 8.23E-06 |
| #9104a-B4 | 1.53E-10 | 2.45E+05 | 3.74E-05 |
| #9104a-B5 | <1.0E-12 | 2.43E+05 | <1.0E-07 |
| #9104a-B6 | 1.14E-09 | 8.29E+04 | 9.41E-05 |
| #9104a-B7 | 1.63E-10 | 2.50E+05 | 4.07E-05 |
| #9104a-B8 | 2.80E-10 | 2.30E+05 | 6.44E-05 |
| #9104a-C1 | 1.39E-10 | 2.71E+05 | 3.76E-05 |
| #9104a-C2 | 1.72E-10 | 3.06E+05 | 5.28E-05 |
| #9104a-C3 | 1.76E-10 | 2.59E+05 | 4.55E-05 |
| #9104a-C4 | 2.03E-10 | 3.17E+05 | 6.42E-05 |
| #9104a-C5 | 1.92E-10 | 3.37E+05 | 6.46E-05 |
| #9104a-C6 | 9.28E-10 | 1.17E+05 | 1.09E-04 |
| #9104a-C7 | 1.52E-10 | 3.00E+05 | 4.56E-05 |
| #9104a-D1 | 9.48E-11 | 2.94E+05 | 2.79E-05 |
| #9104a-D2 | 1.49E-10 | 3.51E+05 | 5.22E-05 |
| #9104a-D3 | 2.29E-10 | 2.94E+05 | 6.73E-05 |
| #9104a-D4 | 2.06E-10 | 2.48E+05 | 5.09E-05 |
| #9104a-D5 | 1.30E-10 | 2.61E+05 | 3.39E-05 |
| #9104a-D6 | 1.07E-09 | 8.12E+04 | 8.70E-05 |
| #9104a-D7 | 1.98E-10 | 2.88E+05 | 5.69E-05 |
| #9104a-D8 | 1.77E-10 | 1.88E+05 | 3.33E-05 |
| #9104a-E1 | 2.89E-10 | 2.15E+05 | 6.21E-05 |
| #9104a-E2 | 2.12E-10 | 2.02E+05 | 4.27E-05 |
| #9104a-E3 | 2.80E-10 | 2.05E+05 | 5.74E-05 |
| #9104a-E4 | 1.97E-10 | 2.10E+05 | 4.15E-05 |
| #9104a-E5 | 2.73E-10 | 2.34E+05 | 6.36E-05 |
| #9104a-E6 | 1.19E-09 | 6.55E+04 | 7.77E-05 |
| #9104a-E7 | 2.11E-10 | 2.18E+05 | 4.59E-05 |
| #9104a-E8 | 3.80E-10 | 1.49E+05 | 5.66E-05 |

As shown in Table 8, the produced antibodies bound to the human ASM protein with a binding affinity of 10⁻¹⁰ to 10^{- 9} M.

## Claims

1. An antibody or antigen-binding fragment thereof that specifically binds to an acid sphingomyelinase (ASM) protein.

2. The antibody or antigen-binding fragment thereof of claim 1, wherein the ASM protein is derived from a mammal.

3. The antibody or antigen-binding fragment thereof of claim 1, wherein the antibody or antigen-binding fragment thereof comprises:
a heavy chain variable region including a heavy chain CDR1 consisting of the amino acid sequence as set forth in SEQ ID NO: 89, a heavy chain CDR2 in which five or fewer amino acids are substituted in a polypeptide consisting of the amino acid sequence as set forth in SEQ ID NO: 90, and a heavy chain CDR3 consisting of the amino acid sequence as set forth in SEQ ID NO: 91; and
a light chain variable region including a light chain CDR1 consisting of the amino acid sequence as set forth in SEQ ID NO: 92, a light chain CDR2, which is a polypeptide consisting of the amino acid sequence as set forth in SEQ ID NO: 93 or in which five or fewer amino acids are substituted in the polypeptide, and a light chain CDR3, which is a polypeptide consisting of the amino acid sequence as set forth in SEQ ID **NO:** 94 or in which two or fewer amino acids are substituted in the polypeptide.

4. The antibody or antigen-binding fragment thereof of claim 3, wherein the heavy chain CDR2 in which five or fewer amino acids are substituted in a polypeptide consisting of the amino acid sequence as set forth in SEQ ID NO: 90 has substitutions of five or fewer amino acids selected from the group consisting of amino acids at positions 2, 4 to 10, 12, 13, and 17 from the N-terminus of the polypeptide.

5. The antibody or antigen-binding fragment thereof of claim 3, wherein the heavy chain CDR2 in which five or fewer amino acids are substituted in a polypeptide consisting of the amino acid sequence as set forth in SEQ ID NO: 90 is a polypeptide consisting of any one of the amino acid sequences as set forth in SEQ ID NOS: 95 to 111.

6. The antibody or antigen-binding fragment thereof of claim 3, wherein the light chain CDR2 in which five or fewer amino acids are substituted in a polypeptide consisting of the amino acid sequence as set forth in SEQ ID NO: 93 has substitutions of four or fewer amino acids selected from the group consisting of amino acids at positions 3 to 7 from the N-terminus of the polypeptide.

7. The antibody or antigen-binding fragment thereof of claim 3, wherein the light chain CDR2 in which five or fewer amino acids are substituted in a polypeptide consisting of the amino acid sequence as set forth in SEQ ID NO: 93 is a polypeptide consisting of any one of the amino acid sequences as set forth in SEQ ID NOS: 112 to 135.

8. The antibody or antigen-binding fragment thereof of claim 3, wherein the light chain CDR3 in which two or fewer amino acids are substituted in a polypeptide consisting of the amino acid sequence as set forth in SEQ ID NO: 94 has substitutions of two or fewer amino acids selected from the group consisting of amino acids at positions 6 to 8 from the N-terminus of the polypeptide.

9. The antibody or antigen-binding fragment thereof of claim 3, wherein the light chain CDR3 in which two or fewer amino acids are substituted in a polypeptide consisting of the amino acid sequence as set forth in SEQ ID NO: 94 is a polypeptide consisting of the amino acid sequence as set forth in SEQ ID NO: 136.

10. A nucleic acid encoding the antibody or antigen-binding fragment thereof of any one of claims 1 to 9.

11. An expression vector comprising the nucleic acid of claim 10.

12. A host cell comprising the nucleic acid of claim 10 or the expression vector of claim 11.

13. A method for producing an antibody or antigen-binding fragment thereof that specifically binds to an ASM protein, the method comprising culturing the host cell of claim 12 to produce the antibody or antigen-binding fragment thereof.
